# EUROPEAN PATENT SPECIFICATION

(11) **EP 3 492 111 B1**
(45) Date of publication and mention of the grant of the patent: **11.05.2022**
(21) Application number: 18208959.9
(22) Date of filing: 28.11.2018
(51) Int. Cl.: A61L 2/18, A61L 2/22, A01N 59/12, A01N 59/00, A01N 65/24

(54) **METHOD FOR DISINFECTION OF ITEMS AND SPACES**
VERFAHREN ZUR DESINFEKTION VON ARTIKELN UND RÄUMEN
PROCÉDÉ DE DÉSINFECTION D'ARTICLES ET D'ESPACES

(30) Priority: 29.11.2017 US 201762591812 P
(43) Date of publication of application: 05.06.2019
(73) Proprietor: Pancheri, Eugene J., Cincinnati, OH 45217 (US)
(72) Inventor: Pancheri, Eugene J., Cincinnati, OH 45217 (US)
(74) Representative: Kuhnen & Wacker Patent- und Rechtsanwaltsbüro PartG mbB

(56) References cited:
- GB-A- 2 257 630
- US-A- 5 772 971
- US-A1- 2007 231 200
- US-A1- 2016 022 850

## Description

### Field of the Invention

The present invention is in the field of disinfection and sterilization methods.

### Background of the Disclosure

There is a need for an inexpensive, effective, yet safe and convenient method to minimize the microbial burden of objects we interact with. This method must not leave behind microbes with resistance to future treatment. And, this need is becoming more compelling as microbes which are resistant to virtually all known antibiotics are becoming more common.

What is needed is a way to kill virtually all microbes in a way that they cannot develop a resistance to the antimicrobial. A potential way to do this would be to utilize ingredients and methods that are relatively safe to humans but are biocidal.

Iodine is a well-known, relatively safe biocide, more recently hydrogen peroxide has been shown to fight microbes and electricity has a biocidal effect. Sunlight, which emits energy in the ultraviolet wavelengths, is also well known for its biocidal properties.

US 2007/0231200 discloses a method and system of treating an instrument after contamination of a surface thereof including the steps of covering the surface with a hydrogen peroxide foam and then subsequently treating the foam with a defoaming agent and a neutralizing agent for hydrogen peroxide.

US 5,772,971 discloses a microbial decontamination system including a chamber for housing items to be decontaminated as well as a source of one or more iodine-containing fluids that are conveyed to the chamber as necessary.

GB 2,257,630 discloses that an organic peroxyacid acting as a broad spectrum biocide is boosted by incorporating an activating amount of a metal selected from iron and copper.

US 2016/0022850 discloses methods and systems for killing or deactivating spores by applying a fluid to a surface containing a spore and applying direct or indirect plasma to the surface for a period of time.

The problem with these safe biocides is that each one individually is not effective against all types of microbes, and several target microbes have developed defense mechanisms against these biocides. However, combinations of two or more of these biocides have proven to work synergistically to enhance each one's effects. In particular, combining a salt of iodine with hydrogen peroxide has been shown to solve several problems with the use of iodine for disinfection. Thus, it is possible to include iodine into the array of safe biocides

Iodine is an exceptional biocide because it is effective against virtually all types of microbes at relatively low dosage. In addition, it has several biocidal mechanisms so there is no known instance of microbes developing resistance to iodine after exposure. Typically, iodine disinfectants have traditionally been used as leave-in-place, on-demand disinfectants. But they leave an unsightly color and other negative traits. Thus, if these negative traits of iodine are not wanted, it must be rinsed-away. When surfaces exceeding tens of square centimeters are to be treated by any of the common commercial forms of iodine, problems arise. There is either so much total iodine transferred to the surface resulting in large unsightly areas, or it requires a lot of post cleanup which is time consuming and expensive.

It is also time consuming and expensive to apply the traditional iodine antiseptics to surfaces exceeding tens of square centimeters. They are traditionally painted on which is time consuming and thus expensive. A cheaper method would be to spray it on. However, that leads to safety issues.

Inhalation of elemental iodine is problematic and more toxic to humans than hydrogen peroxide or iodine salts such as potassium iodide. For example, elemental iodine is considered a poison if dosed internally whereas an iodine salt such as potassium iodide is used as a medicine and a common food additive.

Another safety concern is the droplet size emitted by a sprayer. If it emits droplets smaller in size than about 10 microns it can lead to deep lung penetration which worsens any health concerns. On the other hand, if they are larger than 50 to 80 microns their "hang time" is not sufficient to arrive at distant surfaces because of gravity.

As a result, there is still a need for sterilization and disinfecting methods utilizing iodine that are simultaneously effective, convenient, and safe,

### Summary of the Disclosure

The invention disclosed herein provides an improvement to disinfecting surfaces using iodine chemistry by eliminating instability, solubility and human safety issues associated with forming iodine at any point prior to application on a surface. The disclosed invention provides improved methods for disinfecting surfaces by dispersing iodine producing reactant compounds at very low levels in separate application steps and forming iodine biocides directly on surfaces to be disinfected.

The invention disclosed herein involves applying coatings of a low concentration of a safe iodine salt (such as potassium iodide) and, separately, a low concentration of hydrogen peroxide. Reactions between the iodine salt and hydrogen peroxide produce on surfaces to be treated produces low levels of elemental iodine (I₂) along with very low levels of HOI - which along with I₂ and H₂O₂ provide multiple mechanisms to kill microbes. The low levels of iodine biocide compounds produced by this method does not create the safety, color or odor problems associated with traditional iodine containing disinfectants.

Another aspect of safety is the droplet size emitted by a spray delivery device. The inventive method does not deliver droplets smaller in size than about 10 microns nor does it deliver droplets larger than 50 to 80 microns whose "hang-time" is not sufficient to arrive at distant surfaces because of gravity. "Hang-time" is the amount of time it takes a droplet of a certain diameter to fall one meter under the influence of gravity. Smaller diameter droplets have a considerably longer hang-time than larger diameter droplets.

Both of the above benefits (safety and "hang-time") are enhanced by electrostatics in that charged droplets repel each other (increasing "hang-time") and are attracted to surfaces including the nasal cavity, thus further reducing deep lung penetration.

Another advantage of this method is the ability of all the components to volatilize after the sterilization is complete. The invention disclosed herein provides a way to have an effective iodine leave-in-place disinfectant that does not have residual color, odor or other undesirable effects. Combined with a surface layer that does not exceed 15 microns or preferably 6 microns, the small amount of material applied, and the volatility of the ingredients assures rapid evaporation. Thus, a room or item that has been treated can be quickly returned to service without the need for additional treatments to remove components from treated surfaces.

In one aspect, the disclosure provides a method of disinfecting a surface in need of disinfecting within an area, comprising the steps of: a) dispersing into the area a multiplicity of droplets of a first aqueous composition comprising a first iodine reactant compound that is either a peroxide compound or an iodine salt compound: b) allowing a time sufficient for the first aqueous composition to distribute throughout the area, and to deposit and coalesce into a layer upon the surface: c) dispersing into the area a multiplicity of droplets of a second aqueous composition comprising a second iodine reactant compound that is the other of the first iodine reactant compound, and: d) allowing a second time sufficient for the droplets of the second aqueous composition to deposit onto the coalesced layer of the first aqueous composition, thereby forming iodine and other iodine biocides *in situ* and disinfecting the surface.

In another aspect, an amount of the dispersed first aqueous composition is sufficient to provide the coalesced layer of the first aqueous composition with a substantially uniform thickness of at least about 1 micron and up to about 20 microns.

In another aspect, an amount of the dispersed first aqueous composition is sufficient to provide the coalesced layer of the first aqueous composition with a substantially uniform thickness of at least about 1 micron and up to about 20 microns and the second aqueous composition is dispersed in an amount sufficient to provide a coalesced layer of the second aqueous composition with a substantially uniform thickness of at least about 1 micron and up to about 20 microns.

In another aspect, the amount of the dispersed first aqueous composition is sufficient to provide the coalesced layer of the first aqueous composition with a substantially uniform thickness of at least about 3 microns and up to about 20 microns and the amount of the dispersed second aqueous composition is sufficient to provide as coalesced layer of the second aqueous composition with a substantially uniform thickness of at least about 1 micron sand up to about 20 microns.

In another aspect of the disclosure, at least one of the first aqueous composition or the second aqueous composition can also include other biocide compounds, including but not limited to alcohols and essential oils.

In another aspect of the disclosure, the method further includes the step of illuminating either or both of the first and second reactant compound and/or the surface on which they deposit within the area with a wavelength consisting essentially of ultraviolet light.

In another aspect of the disclosure, the multiplicity of droplets of the first aqueous composition is electrostatically charged.

In another aspect, the multiplicity of electrostatically charged droplets of the first aqueous composition are negatively charged or the multiplicity of electrostatically charged droplets of the first aqueous composition are positively charged.

In another aspect of the disclosure the charge polarity of the sprayer when it sprays the first and the second compound is optimized to provide the most desirable reaction of the first and second compounds.

In another aspect of the disclosure, the multiplicity of droplets of the second aqueous composition is electrostatically charged with the opposite polarity of the first aqueous composition.

In another aspect, the multiplicity of droplets of the second aqueous composition is electrostatically charged and the multiplicity of electrostatically charged droplets of the second aqueous composition are negatively charged or the multiplicity of electrostatically charged droplets of the second aqueous composition are positively charged.

In another aspect of the disclosure, the surface in need of disinfecting is grounded.

In another aspect, the disclosure provides a method of disinfecting a surface in need of disinfecting within an area, comprising the steps of: a) dispersing into the area a multiplicity of electrostatically-charged droplets of a first aqueous composition comprising a biocidal compound selected from the group consisting of: i) a peroxide compound or an iodine salt, ii) an alcohol, iii) an essential oil; b) allowing a first time sufficient for the first aqueous composition to distribute throughout the area, and to deposit and coalesce into a layer upon the surface; c) dispersing into the area a multiplicity of droplets of a second aqueous composition comprising a different one of the biocidal compound, and; d) allowing a second time sufficient for the droplets of the second aqueous composition to deposit onto the coalesced layer of the first aqueous composition, thereby disinfecting the surface.

In another aspect, the disclosure provides a method of disinfecting a surface, comprising the steps of: a) spraying electrostatically a first aqueous composition comprising a peroxide compound, toward and into contact with the surface; b) spraying a second aqueous composition comprising an iodine salt toward and into contact with the first aqueous composition on the surface, and; c) allowing the second aqueous liquid composition to contact the first aqueous liquid composition, thereby forming iodine biocides *in situ* and disinfecting the surface.

In another aspect, the disclosure provides a method of disinfecting a surface in need of disinfecting within an area containing ambient air, comprising the steps of: a) heating a first aqueous composition comprising a peroxide compound to produce a vapor comprising the peroxide compound in the ambient air; b) allowing a first time sufficient for the vapor comprising the peroxide compound to distribute throughout the area, and to cool, condense and deposit into a liquid layer upon the surface, the liquid layer comprising the peroxide compound; c) heating a second aqueous composition comprising an iodine salt compound, and; d) allowing a second time sufficient for the iodine salt compound to distribute throughout the area, and to cool and deposit the iodine salt compound onto the liquid layer comprising the peroxide compound, thereby forming an iodine biocide *in situ and* disinfecting the surface.

In another aspect of the disclosure, the first aqueous composition is heated at about 250° C.

In another aspect of the disclosure, the first aqueous composition and the second aqueous composition are cooled to about 55° C to condense and deposit onto surfaces within the area to be disinfected.

In another aspect of the disclosure, the first aqueous composition and the second aqueous composition are comprised of food-grade components.

In another aspect, the disclosure provides a method of disinfecting a surface in need of disinfecting within an area containing ambient air, comprising the steps of: a) introducing a first aqueous composition comprising a peroxide compound into a first hot gaseous stream to produce a vapor comprising the peroxide compound, and discharging the first hot gaseous stream comprising the peroxide compound vapor into the ambient air; b) allowing a first time sufficient for the vapor comprising the peroxide compound to distribute throughout the ambient air of the area, and to cool, condense and deposit into a liquid layer upon the surface, the liquid layer comprising the peroxide compound; c) introducing a second aqueous composition comprising an iodine salt compound into a second hot gaseous stream and discharging the second hot gaseous stream comprising the iodine salt compound into the ambient air, and; d) allowing a second time sufficient for the iodine salt compound to distribute throughout the ambient air of the area, and to cool and deposit the iodine salt compound onto the first liquid layer comprising the peroxide compound, thereby forming an iodine biocide *in situ* and disinfecting the surface.

### Detailed Description of the Invention

The present disclosure relates to a method for sterilizing rooms, areas, and surfaces within those areas; particularly by generating iodine biocides on those targets *in situ* by applying iodine biocide reactant compounds in two or more separate applications. The method described herein is safer because the iodine biocides are formed directly on the target only after all reaction components have been applied.

The term, "health care surface" refers to a surface of a surface of an instrument, a device, a cart, a cage, furniture, a structure, a building, or the like that is employed as part of a health care activity.

As used herein, the term, *"in situ,"* refers to the production of an iodine biocide directly on the target surface, after application of two separate compositions containing iodine reactant compounds. This is distinct from other sterilization or disinfection methods that utilize the term *in situ* to describe the location at which two or more reaction mixtures are first mixed in a reaction vessel to form an iodine biocide, before subsequently applying the iodine biocide onto a surface or area.

As used herein, the term, "instrument," refers to the various medical or dental instruments or devices that can benefit from cleaning with a composition according to the present disclosure.

As used herein, the term "microorganism" refers to any noncellular or unicellular (including colonial) organism. Microorganisms include all prokaryotes. Microorganisms include bacteria (including cyanobacteria), spores, lichens, fungi, protozoa, virinos, viroids, viruses, phages, and some algae. As used herein, the term "microbe" is synonymous with microorganism.

As used herein, the phrase, "iodine salt", refers to any salt of iodine that is capable of forming an iodine biocide that is effective as a disinfecting agent.

As used herein, the term, "iodine biocide compound" refers to any compound that can react with an iodine salt to form an iodine biocide, including but not limited to hydrogen peroxide, metal peroxides, and ozone.

As used herein, the term "polyhydric alcohol" refers to an alcohol that has two or more hydroxyl groups. Polyhydric alcohols suitable for use in the aqueous compositions include but are not limited to sugars, sugar alcohols, and non-aliphatic polyhydric alcohols such as phenols.

Concentrations, dimensions, amounts, and other numerical data may be presented herein in a range format. It is to be understood that such range format is used merely for convenience and brevity.

In accordance with these definitions, several methods for disinfecting target surfaces within an area by forming iodine biocides on those surfaces *in situ* are provided. The potential applications for these methods are extraordinarily diverse, including but not limited to disinfecting animal living spaces, food products and processing surfaces, health care surfaces and instruments, laboratories, restrooms, vehicles, schools, offices, public transportation, industrial facilities, and countless other areas and surfaces. This disclosure overcomes the deficiencies associated with direct delivery of iodine for sterilization, particularly with regard to the instability, insolubility and safety of iodine biocides.

This disclosure harnesses the power of iodine chemistry to disinfect target surfaces while utilizing ingredients that are safe and have a very long shelf life and that anyone can obtain at their local grocery store or pharmacy.

Without being limited by theory, the antimicrobial action of iodine is quick and effective at low concentrations, and thus it is used in operating theatres. It is believed to penetrate into microorganisms and attack particular amino acids (such as cysteine and methionine), nucleotides, and fatty acids, ultimately resulting in cell death. It also has an antiviral action. It may also destabilize membrane fatty acids by reacting with unsaturated carbon bonds.

It is believed that peroxides are effective as disinfectants because they are powerful oxidizing agents that can irreversibly damage proteins and DNA within microorganisms. Additionally, peroxides can reactivate iodine if it is reduced to iodide by oxidizing it. Thus, lower levels of iodine are required because peroxides can recycle iodide back to the iodine active state.

Alcohols are also believed to be effective biocides denaturing cell walls of microbes making it harder for them to control their cell contents. This mechanism would also reduce a microbe's ability to resist the penetration of iodine and peroxides. It also helps other biocides work by lowering the surface tension of water.

Used together, the various components of this disclosure work together to produce an exceptional microbe kill by a large number of differing mechanisms. This makes it virtually impossible for a microbe to acquire immunity.

Essentially, this disclosure provides a method for disinfecting surfaces within an area that needs disinfecting, the method comprising the steps of: dispersing into the area a multiplicity of droplets of a first aqueous composition comprising a first iodine biocide reactant compound; allowing a time sufficient for the first aqueous composition to distribute throughout the area, and to deposit and coalesce into a layer about the surfaces; dispersing a multiplicity of droplets of a second aqueous composition comprising a second iodine biocide reactant compound; and allowing a second time sufficient for the second aqueous composition to deposit onto the coalesced layer of the first aqueous composition, thereby forming an iodine biocide *in situ* and disinfecting the surfaces.

So long as an iodine biocide is formed only on the surface to be disinfected, the effectiveness of the method is independent of the order in which the iodine biocide reactant compounds are dispersed. Thus, the first iodine biocide reactant compound can either be an iodine salt compound or a peroxide compound, so long as the second iodine biocide reactant compound is the opposite compound of that chosen to be the first iodine biocide reactant compound.

The peroxide compound present in either aqueous composition is any compound that can react with an iodine salt to form an iodine biocide. Generally, these will include but not be limited to hydrogen peroxide, metal peroxides, or ozone. In some embodiments, the peroxide compound is hydrogen peroxide. The peroxide compound may be present in an aqueous composition at concentrations ranging from about 0.1% to about 10% by weight.

The iodine salt compound present in either aqueous composition is any iodine salt that can effectively form an iodine biocide by reacting with a peroxide compound. Non-limiting examples of compounds which can be used include hydrogen iodide, sodium iodide, and potassium iodide. The iodine salt may be present in an aqueous composition at concentrations ranging from about 0.01% to about 10% by weight.

In some embodiments of the disclosure, the disinfectant methods described above for generating iodine biocides on surfaces to be disinfected can be used for a variety of user-identified biocidal and/or anti-microbial purposes, including antimicrobial, bleaching, or sanitizing applications.

In some embodiments, the iodine biocides generated according to the method of the present disclosure are effective for killing one or more of the pathogenic bacteria associated with a health care surfaces and instruments including but not limited to, *Salmonella typhimurium, Staphylococcus aureus, Salmonella choleraesurus,* Pseudomonas aeruginosa, *Escherichia coli,* Mycobacteria, yeast, and mold. In other embodiments, the generated iodine biocides are also effective in domestic or industrial applications and can be applied in a variety of areas including but not limited to kitchens, bathrooms, factories, hospitals, dental offices, restaurants, laundry or textile services, animal stalls and food processing plants.

Furthermore, the iodine biocides generated according to the method of the present disclosure are effective against a wide variety of microorganisms, such as Gram- positive organisms (*Listeria monocytogenes* or *Staphylococcus aureus*), Gram-negative organisms (*Escherichia coli* or *Pseudomonas aeruginosa*)*,* catalase-positive organisms (*Micrococcus luteus* or *Staphylococcus epidermidis*), or sporulent organisms *(Bacillus subtilis).*

The disclosure is illustrated by the following examples:

### Examples

The following example illustrates the embodiments of the disclosure that are presently best known. However, it is to be understood that the following is only illustrative of the application of the principles of the present disclosure. Numerous modifications and alternative compositions, methods, and systems may be devised by those skilled in the art without departing from the spirit and scope of the present disclosure.

### Example 1: Closed-System Electrospray Distribution and Log-Kill Studies

A study was conducted in accordance with embodiments of the present disclosure to determine the antimicrobial effect of a method where an iodine biocide is formed on target surfaces within a 1 cubic meter translucent plastic cube *in situ* by applying two separate aqueous compositions, one containing potassium iodide and one containing hydrogen peroxide. The following ingredients are included in approximate amounts:
First Aqueous Composition:
   0.64% (w/w) potassium iodide
   15% (w/w) Ethanol
   0.003% (w/w) Cinnamon Oil
   84.357% (w/w) Distilled Water
Second Aqueous Composition:
   5.25% (w/w) Hydrogen Peroxide
   15% (w/w) Ethanol
   79.75% (w/w) Distilled Water

To simultaneously evaluate whether an electrospray device would evenly distribute an aqueous composition including an iodine biocide reactant compound, the first aqueous composition is applied using a Hurricane ES^{™} Portable Electrostatic Aerosol Applicator. Two analytical balances are placed inside the cube and connected to a computer station configured to collect and record mass measurements as a function of time. On each balance, a 1000 square centimeter plastic sheet is placed on the balance weighing pan. The position of each balance is staggered to be in different positions along the x, y, and z axes in relation to the position of the electrostatic sprayer. The second aqueous composition is applied using a hand sprayer.

Cultures from commercially-available strains of three species of bacteria-*Bacillus subtilis, Micrococcus luteus,* and *Staphylococcus epidermis-are* selected for the log-kill studies because they possess several known defense mechanisms to common biocides while at the same time having different physical properties from each other. Sterilized, pre-poured agar plates are used as growth media to produce colonies of each bacteria. Eight plates are inoculated for each species. Of those 8 plates, 4 plates are exposed to the aqueous compositions that contain the iodine biocide reactant compounds, and 4 are held out as controls. Plates are inoculated using the standard T-method of streaking for log-kill studies, where the concentration of bacteria in the fourth quadrant of the plate is approximately 1,000,000X diluted with respect to the first quadrant. The test plates for each species are then placed inside the cube. After the cube is closed, the lids are opened. Control plates are sealed with tape.

The first aqueous composition is then applied to the entire cube by electrospray for 30 seconds with a set particle size of about 15 to 60 microns. During the application, mass measurements from the two balances are collected and recorded by the computer. The time of application is selected to provide a 3 microns thick coating within the treatment space as measured by the balances. After about 1 minutes, the second aqueous composition is applied using a hand sprayer, and the entire system is untouched for another 5 minutes. Lids are replaced on each of the test plates inside the cube before being brought out into the ambient environment, where they are sealed with tape. The sealed test plates and the control plates are then incubated at about 28°C and inspected after 1, 2, and 4 days.

The results of the tests are provided as follows:

**TABLE 1**

| |
|---|
| Electrospray Distribution |
| Mass - First Aqueous Composition (g) |
| Balance A (with 1000 cm^2 plate) 0.201 |
| Balance B (with 1000 cm^2 plate) 0.195 |

**TABLE 2**

| Presence of colonies after 1 day (+ or -) | | | | |
|---|---|---|---|---|
| Plate Number | | *B. subtilis* | *M. luteus* | *S. epidermis* |
| 1 | - | - | - | |
| 2 | - | - | - | |
| 3 | - | - | - | |
| 4 | - | - | - | |

**TABLE 3**

| | | | | |
|---|---|---|---|---|
| Presence of colonies after 2 days (+ or -) | | | | |

| Plate Number | | *B. subtilis* | *M. luteus* | *S. epidermis* |
|---|---|---|---|---|
| 1 | - | - | - | |
| 2 | - | - | - | |
| 3 | - | - | - | |
| 4 | - | - | - | |

**TABLE 4**

| Presence of colonies after 4 days (+ or -) | | | | |
|---|---|---|---|---|
| Plate Number | | *B. subtilis* | *M. luteus* | *S. epidermis* |
| 1 | - | - | - | |
| 2 | - | - | - | |
| 3 | - | - | - | |
| 4 | - | - | - | |

The mass of the first aqueous composition deposited on balance A and balance B indicates a difference of 0.006 grams which is only a 3% difference. In combination with a qualitative observation that the inside surfaces of the cube appear to be equally coated with liquid, it is believed that the electrospray evenly distributes the first aqueous composition within the cube.

All controls produce the expected results, with positive control plates not treated with aqueous compositions containing iodine biocide reactant compounds showing characteristic growth for each organism. Over the 16 control plates, there is an average of 4 or 5 colonies in the fourth quadrant of the plate, indicating that there are 45,000,000 colonies in the initial inoculation.

Therefore, the lack of colonies on the test plates, coupled with the approximately 45,000,000 colonies observed on the control plates, indicates that the method is effective to at least a log-6 kill rate, representing a kill of 99.9999% of the bacteria originally present on the plate.

Example II: Fifteen Petri dishes containing nutrient agar were streaked with *Bacillus subtilus* in three zones in the traditional manner. Five of the Petris were sealed after streaking (control). Five Petris were sprayed with a solution containing 2 ppm KI and 10% ethanol, sufficiently to wet the surface, and then sealed. And, five Petris were initially sprayed as before, and then subsequently sprayed with a solution containing 5% hydrogen peroxide and 2.5% isopropanol, and then sealed.

After incubating all 15 Petri dishes at 40° C for three days, the number of colony forming units was estimated as follows: about 10k for the control; about 6k for the KI control and "0" for the double spray test.

While particular embodiments of the disclosure have been described, the disclosure can be further modified.

Those skilled in the art will recognize, or be able to ascertain using no more than routine experimentation, numerous equivalents to the specific procedures, embodiments, and examples described herein. The invention is defined by the appended independent claims. Any information falling outside the scope of the claims is for explanation only.

## Claims

1. A method of disinfecting a surface in need of disinfecting, within an area, said method comprising:
dispersing into said area a multiplicity of droplets of a first aqueous composition comprising a first reactant compound that is either a peroxide compound or an iodine salt compound, the droplets of the first aqueous composition being no smaller in size than about 10 microns and no larger in size than about 80 microns;
allowing a time sufficient for said first aqueous composition to distribute throughout the area and to deposit and coalesce into a layer upon said surface;
dispersing into said area a multiplicity of droplets of a second aqueous composition comprising a second reactant compound that is the other of the first reactant compound, the droplets of the second aqueous composition being no smaller in size than about 10 microns and no larger in size than about 80 microns; and,
allowing a second sufficient time for the droplets of the second aqueous composition to deposit onto the coalesced layer of the first aqueous composition to form a reaction layer thereby forming a biocide reactant compound of iodine *in situ* in the reaction layer and disinfecting said surface.

2. The method of claim 1 wherein an amount of the dispersed first aqueous composition is sufficient to provide the coalesced layer of the first aqueous composition with a substantially uniform thickness of at least about 1 micron and up to about 20 microns.

3. The method of claim 2 wherein the amount of the dispersed second aqueous composition is sufficient to provide a coalesced layer of the second aqueous composition with a substantially uniform thickness of at least about 1 micron and up to about 20 microns.

4. The method of claim 3 wherein the amount of the dispersed first aqueous composition is sufficient to provide the coalesced layer of the first aqueous composition with a substantially uniform thickness of at least about 3 microns and up to about 20 microns.

5. The method of claim 1 wherein the multiplicity of droplets of the first aqueous composition are electrostatically charged droplets.

6. The method of claim 5 wherein the multiplicity of electrostatically charged droplets of the first aqueous composition are negatively charged.

7. The method of claim 5 wherein the multiplicity of electrostatically charged droplets of the first aqueous composition are positively charged.

8. The method of claim 1 wherein the multiplicity of droplets of the second aqueous composition are electrostatically charged droplets.

9. The method of claim 8 wherein the multiplicity of electrostatically charged droplets of the second aqueous composition are negatively charged.

10. The method of claim 8 wherein the multiplicity of electrostatically charged droplets of the second aqueous composition are positively charged.

11. The method of claim 1 wherein the first aqueous composition comprises about 0.01% to about 1.0% by weight potassium iodide.

12. The method of claim 1 wherein the second aqueous composition comprises about 3% to about 7% by weight hydrogen peroxide.

## Patentansprüche

1. Verfahren zum Desinfizieren einer Oberfläche, die eine Desinfektion in einem Bereich benötigt, wobei das Verfahren aufweist:
Verteilen einer Mehrzahl an Tröpfchen einer ersten wässrigen Zusammensetzung in dem Bereich, die eine erste Reaktionsverbindung aufweist, die entweder eine Peroxidverbindung oder eine Jodsalzverbindung ist, wobei die Tröpfchen der ersten wässrigen Zusammensetzung nicht kleiner sind als ungefähr 10 Mikron und nicht größer sind als ungefähr 80 Mikron;
Abwarten für einen ausreichenden Zeitraum, so dass sich die erste wässrige Zusammensetzung in dem Bereich verteilen kann und sich an einer Schicht auf der Oberfläche absetzt und in dieser gerinnt;
Verteilen einer Mehrzahl an Tröpfchen einer zweiten wässrigen Zusammensetzung in dem Bereich, die eine zweite Reaktionsverbindung aufweist, die die entsprechende andere der ersten Reaktionsverbindung ist, wobei die Tröpfchen der zweiten wässrigen Zusammensetzung nicht kleiner sind als ungefähr 10 Mikron und nicht größer sind als ungefähr 80 Mikron; und
Abwarten für einen zweiten ausreichenden Zeitraum, so dass sich die Tröpfchen der zweiten wässrigen Zusammensetzung auf der geronnenen Schicht der ersten wässrigen Zusammensetzung absetzen, so dass eine Reaktionsschicht entsteht, wodurch sich eine Biozid-Reaktionsverbindung aus Jod *in situ* in der Reaktionsschicht bildet und die Oberfläche desinfiziert.

2. Verfahren nach Anspruch 1, wobei eine Menge der verteilten ersten wässrigen Zusammensetzung ausreichend ist, um die geronnene Schicht der ersten wässrigen Zusammensetzung mit einer im Wesentlichen gleichmäßigen Dicke von mindestens ungefähr 1 Mikron und bis zu ungefähr 20 Mikron zu versehen.

3. Verfahren nach Anspruch 2, wobei die Menge der verteilten zweiten wässrigen Zusammensetzung ausreichend ist, um die geronnene Schicht der zweiten wässrigen Zusammensetzung mit einer im Wesentlichen gleichmäßigen Dicke von mindestens ungefähr 1 Mikron und bis zu ungefähr 20 Mikron zu versehen.

4. Verfahren nach Anspruch 3, wobei die Menge der verteilten ersten wässrigen Zusammensetzung ausreichend ist, um die geronnenen Schicht der ersten wässrigen Zusammensetzung mit einer im Wesentlichen gleichmäßigen Dicke von mindestens ungefähr 3 Mikron und bis zu ungefähr 20 Mikron zu versehen.

5. Verfahren nach Anspruch 1, wobei die Mehrzahl an Tröpfchen der ersten wässrigen Zusammensetzung elektrostatisch geladene Tröpfchen sind.

6. Verfahren nach Anspruch 5, wobei die Mehrzahl an elektrostatisch geladenen Tröpfchen der ersten wässrigen Zusammensetzung negativ geladen ist.

7. Verfahren nach Anspruch 5, wobei die Mehrzahl an elektrostatisch geladenen Tröpfchen der ersten wässrigen Zusammensetzung positiv geladen ist.

8. Verfahren nach Anspruch 1, wobei die Mehrzahl an Tröpfchen der zweiten wässrigen Zusammensetzung elektrostatisch geladene Tröpfchen sind.

9. Verfahren nach Anspruch 8, wobei die Mehrzahl an elektrostatisch geladenen Tröpfchen der zweiten wässrigen Zusammensetzung negativ geladen ist.

10. Verfahren nach Anspruch 8, wobei die Mehrzahl an elektrostatisch geladenen Tröpfchen der zweiten wässrigen Zusammensetzung positiv geladen ist.

11. Verfahren nach Anspruch 1, wobei die erste wässrige Zusammensetzung ungefähr 0,01 bis ungefähr 1,0 Gewichtsprozent an Kaliumjodid aufweist.

12. Verfahren nach Anspruch 1, wobei die zweite wässrige Zusammensetzung ungefähr 3 bis ungefähr 7 Gewichtsprozent an Wasserstoffperoxid aufweist.

## Revendications

1. Procédé pour désinfecter une surface qui doit être désinfectée dans un lieu, ce procédé consistant à :
- disperser dans le lieu une multiplicité de gouttelettes d'une première composition aqueuse comprenant un premier composant réactif qui est soit un composant peroxyde, soit un composant de sel d'iode, les gouttelettes de cette première composition aqueuse étant de dimensions supérieures à environ 10 microns et inférieures à environ 80 microns,
- laisser pendant une durée suffisante cette première composition aqueuse se répartir dans le lieu et se déposer et coalescer en une couche sur la surface,
- disperser dans le lieu, une multiplicité de gouttelettes d'une seconde composition aqueuse comprenant un second composant réactif qui est différent du premier composant réactif, les gouttelettes de cette seconde composition aqueuse ayant une dimension supérieure à environ 10 microns et inférieure à environ 80 microns, et
- laisser pendant une seconde durée suffisante, les gouttelettes de la seconde composition aqueuse se déposer sur la couche de coalescence de la première composition aqueuse pour former une seconde couche de réaction en formant un composant réactif, biocide, d'iode in situ dans la couche de réaction et désinfectant la surface.

2. Procédé selon la revendication 1,
selon lequel
une quantité de la première composition aqueuse dispersée est suffisante pour obtenir une couche coalescente de la première composition aqueuse d'épaisseur pratiquement uniforme d'au moins 1 micron jusqu'à environ 20 microns.

3. Procédé selon la revendication 2,
selon lequel
la quantité de la seconde composition aqueuse dispersée est suffisante pour obtenir une couche coalescente de la seconde composition aqueuse ayant une épaisseur pratiquement uniforme d'au moins 1 micron et allant jusqu'à 20 microns.

4. Procédé selon la revendication 3,
selon lequel
la quantité de la première composition aqueuse dispersée est suffisante pour obtenir la couche coalescée de la première composition aqueuse ayant une épaisseur pratiquement uniforme d'au moins 3 microns allant jusqu'à environ 20 microns.

5. Procédé selon la revendication 1,
selon lequel
la multiplicité des gouttelettes de la première composition aqueuse est constituée de gouttelettes à charge électrostatique.

6. Procédé selon la revendication 5,
selon lequel
la multiplicité des gouttelettes à charge électrostatique de la première composition aqueuse est à charge négative.

7. Procédé selon la revendication 5,
selon lequel
la multiplicité des gouttelettes à charge électrostatique de la première composition aqueuse est à charge positive.

8. Procédé selon la revendication 1,
selon lequel
la multiplicité des gouttelettes de la seconde composition aqueuse est composée de gouttelettes à charge électrostatique.

9. Procédé selon la revendication 8,
dans lequel
la multiplicité de gouttelettes à charge électrostatique de la seconde composition aqueuse correspond à une charge négative.

10. Procédé selon la revendication 8,
selon lequel
la multiplicité des gouttelettes à charge électrostatique de la seconde composition aqueuse est à une charge positive.

11. Procédé selon la revendication 1,
selon lequel
la première composition aqueuse contient entre environ 0,01 % et 1.0 % pondéraux d'iodure de potassium.

12. Procédé selon la revendication 1,
selon lequel
la seconde composition aqueuse contient entre environ 3 % et 7 % en poids de peroxyde d'hydrogène.
